(19) Europäisches Patentamt

European Patent Office

Office européen des brevets

(11) Publication number: **0 191 061**
**B1**

(12) **EUROPEAN PATENT SPECIFICATION**

(45) Date of publication of patent specification: **08.08.90**

(21) Application number: **85904013.1**

(22) Date of filing: **15.08.85**

(86) International application number:
**PCT/GB85/00364**

(87) International publication number:
**WO 86/01532 13.03.86 Gazette 86/06**

(51) Int. Cl.⁵: **C 12 N 15/00, C 12 P 21/02, A 61 K 37/54, C 07 H 21/04, C 07 K 13/00**

(54) POLYPEPTIDE AND POLYPEPTIDE COMPOSITION.

(30) Priority: **21.08.84 GB 8421210**

(43) Date of publication of application:
**20.08.86 Bulletin 86/34**

(45) Publication of the grant of the patent:
**08.08.90 Bulletin 90/32**

(84) Designated Contracting States:
**AT BE CH DE FR GB IT LI LU NL SE**

(56) References cited:
**GB-A-2 091 268**

**Chemical Abstracts, volume 71, nr. 23, 8 December 1969, Columbus, Ohio, (US) Verger, R. et al.: "Purification from porcine pancreas of two molecular species with lipase activity", page 26, abstract number 109248d**

**Chemical Abstracts, volume 96, nr. 11, 15 March 1982, Columbus, Ohio, (US) De Caro, J. et al.: "Porcine pancreactic lipase. Completion of the primary structure", page 263 abstract number 81865t**

(73) Proprietor: **CELLTECH LIMITED**
**244-250 Bath Road**
**Slough Berkshire SL1 4DY (GB)**

(72) Inventor: **LOWE, Peter, Anthony**
**9 Melrose Avenue Reading**
**Berkshire RG6 2BN (GB)**

(74) Representative: **Votier, Sidney David et al**
**CARPMAELS & RANSFORD 43, Bloomsbury Square**
**London WC1A 2RA (GB)**

(56) References cited:

**Chemical Abstracts, volume 96, nr. 7, 15 February 1982, Columbus, Ohio, (US) De Caro, Alain et al.: "Comparative studies of human and porcine pancreatic lipases: N-terminal sequences, sulfhydryl groups and interfacial activity",see page 255 abstract number 48236r**

Courier Press, Leamington Spa, England.

# EP 0 191 061 B1

(56) References cited:

Chemical Abstracts, volume 97, nr. 25, 20 Decemnber 1982, Columbus, Ohio, (US) Vasil, Michael L. et al.: "Cloning of a phosphate-regulation hemolysin gene (phospholipase C) from Pseudomonas aeruginosa", page 240 abstract number 209601u

GASTROENTEROLOGY Vol. 60, no. 1, 1971, pp. 1-15; B.B.A., vol. 959, (1988) H-247.252

Chemical Abstract, vol. 97, no. 21, 22Nov.1982, Columbus, Ohio, US Tiruppathi, C. et al.: " Purification and properties of an acid lipase from human gastric juice ", page 371, abstract 177584x

EP 0 191 061 B1

**Description**

This invention relates to a polypeptide and a composition comprising the polypeptide. The polypeptide may be produced by the technique of recombinant DNA biotechnology.

The lipolysis of dietary fat is an important feature of the digestive systems of higher animals. The digestive process is made possible by enzyme catalysed hydrolysis of triglycerides to produce a mixture of monoglycerides, diglycerides, glycerol and free fatty acids as the fats pass through the digestive tract. The hydrolysis products are able to pass through the epithelial membrane of mucosal cells lining the gut. Once absorbed they are used to resynthesise triglycerides which are incorporated in chylomicrons. Chylomicrons are transported by the lymph system away from the site of absorption. The enzymes carrying out triglyceride hydrolysis are termed lipases and are secreted into the gastrointestinal tract (Desnuelle, P (1972)). The Enzymes Vol. VII, 3rd Edition, Acad. Press, New York and London, and Verger, R. (1980) Methods in Enzymology *64*, 340—392).

An enzyme involved in triglyceride hydrolysis is pancreatic lipase (EC 3.1.1.3). The pig is a convenient source of enzyme and pig pancreatic lipase has been extensively studied. It is present at approximately 2.5% of the total proteins in pig pancreatic juice, and has been purified to homogeneity (Verger, R. *et al* (1969) Biochem Biophys Acta *188*, 272—282). The complete amino acid sequence of the enzyme has been determined (De Caro, J. *et al* Biochem Biophys Acta *671*, 129—138). The enzyme comprises a protein portion of 449 amino acids (MW 49859) with a carbohydrate portion (MW about 2000) attached to an Asn residue at position 166 in the amino acid sequence. The total molecular weight of the enzyme is therefore approximately 52000. The catalytic activity of pancreatic lipase is complex since there exists a phase separation between the soluble enzyme and the insoluble triglyceride substrate. In order for the emzyme to interact with the substrate a coenzyme known as colipase is necessary. Colipase is a low molecular weight protein which adsorbs to the solution/lipid interface and then acts as an anchor for lipase, allowing interaction between the enzyme and its lipid substrate.

Pancreatic lipase may be assayed by a variety of techniques (see Desnuelle and Verger as above) involving the measurement of the disappearance of the triglyceride or the appearance of free fatty acid or glycerol. Radioactive labelling, proton release during hydrolysis, and the effect of lipase on the physical properties of a lipid monolayer may also be used to assay lipase activity. In all cases pancreatic lipase is optimally active in the neutral-alkaline pH range (i.e. pH7—pH9) (see Verger *et al* as above). The enzyme is highly sensitive to acid pH and is rapidly inactivated at low pH.

A number of lipid malabsorption illnesses of the human body are characterised by reduced levels of pancreatic lipase secretion.

About eighty percent of individuals suffering from cystic fibrosis suffer from pancreatic insufficiency. Pancreatic lipase insufficiency manifests itself shortly after birth and continues throughout the lifetime of the patient.

Pancreatitis is a condition in which the action of the pancreas is impaired. Pancreatitis often develops in chronic alcoholics who, as a result, suffer from malabsorption of fats and consequent malnutrition.

A developing foetus is dependent upon high carbohydrate nutrition, and has a poorly developed pancreatic function producing low levels of pancreatic lipase. At birth the high carbohydrate nutrition of the foetal period is replaced by a high fat diet as the infant begins to take its mothers milk. Fats account for about half an infant's calorie input. The pancreatic function, even in infants that are carried to full term, is not fully productive and infants, especially those born prematurely, may suffer from inadequate fat digestion leading to appreciable steatorrhea (passage of undigested fat in the faeces) and to a resulting loss of energy.

The present treatment of patients suffering from a deficiency of pancreatic lipase is the oral administration of very large doses of a crude preparation of pig pancreas enzyme. Pancreatic lipase is inactivated by low pH. Such conditions are prevalent in the stomach, with the result that orally administered pancreatic lipase is virtually completely inactivated on the passage through the stomach to the gut. Therefore this effect cannot be completely overcome by the use of large doses of enzyme. The large doses administered are inadequate for most patients, are impure and unpalatable. Certain tablets have been formulated which pass through the acid regions of the stomach and discharge the enzyme only in the relatively alkaline environment of the jejunum (Gow. R. *et al* (1981) the Lancet Vol. II *8255*, 1070—1074). However, many patients suffering from pancreatic disorders have an abnormally acid jejunum and such tablets may fail to discharge the enzyme and may therefore be ineffective.

There is a great need for a preparation of a lipase which may be orally administered to patients suffering from a deficiency of pancreatic lipase.

Published European patent application No. EP—A1—0131418 describes one such preparation comprising lingual lipase, an acid stable lipase originating from the tongue and capsule of carrying out lipolysis in the lumen of the stomach. The present invention provides a preparation comprising a gastric lipase, a lipase originating from stomach tissue and also capable of carrying out lipolysis in the stomach lumen.

Prior to the present invention only preliminary studies on the existence and enzymological properties of human gastric lipase had been carried out. A review (Desnuelle, P. (1971), The Enzymes, Vol. VII, 3rd Edition, Acad. Press, NY and London) stated that "the case of gastric lipase is not yet firmly established".

3

A series of reports by Szafran, Z. *et al* (Enzyme, (1983) *30*, 115—121; Digestion (1978) *18*, 310—318; and Enzyme (1978), *23* 187—193) indicate that gastric mucosa secretes an acid stable lipase. The experimental basis of this rests on comparative zymograms (polyacrylamide gels of protein extracts which are stained for enzymic activity) of gastric mucosa tissue and gastric aspirates together with studies on the apparent co-secretion of the pepsin, hydrogen ions and lipase activity from the stomach mucosa after treatment of the patient with pentagastrin (pentagastrin stimulates secretion of fluid and enzymes from the gastric mucosa). Both gastric juice and stomach mucosa produce closely similar zymogram patterns when stained for lipase activity. However, zymograms of duodenal tissue are markedly different from stomach mucosal tissue indicating that duodenal tissue does not secrete this gastric lipase. Measurement of pepsin, hydrogen ions and lipase activity appearing in human stomach aspirates after continuous administration of pentagastrin in graded doses showed an apparent coupled secretion. No information on the protein chemistry of the lipase from gastric mucosa was provided in this work.

A lipase has been purified to homogeneity from human gastric aspirates (the liquid content of the stomach lumen (see Tiruppathi, C. and Balsubramanian, K. A. Biochim, Biophys. Acta. (1982) *712*, 692—697).

The enzyme has the following properties:—

a. Molecular weight approximately 45,000,

b. Capable of carrying out lipolysis under acidic conditions (between pH 3.5—6.5).

Hence, the enzyme resembled lingual lipase and its origin was attributed to the lingual serous glands by these authors. However, from the above, it is also possible that an unknown fraction of this enzyme originated from the human gastric mucosa. The lipase present in human gastric aspirates may therefore be a mixture of lingual and gastric enzymes. We have now shown that human gastric mucosa secretes a lipase. This human gastric lipase has been shown to be generally similar in chemical composition to lingual lipase but to differ in particular structural respects.

All the work reported above on gastric lipase has been exclusively of an academic nature, and no suggestion has been made of using gastric lipase for the treatment of lipase deficiency. We believe that gastric lipase can be so used, but it is only economic to do so if gastric lipase can be produced on a large scale and at relatively little expense. It is clearly impractical to do this by extraction from animal or human tissue.

We provide gastric lipase in such commercially worthwhile amounts by producing it, in accordance with the invention, using recombinant DNA techniques.

According to a first aspect of the present invention we provide a gastric lipase protein for use in the treatment of lipase deficiency.

As used herein the term "gastric lipase protein" denotes an authentic mammalian gastric lipase or an authentic mammalian gastric lipase modified or substituted to provide a functionally equivalent protein. The gastric lipase protein may, for example, comprise a mammalian gastric lipase protein with an N-terminal methionine amino acid residue (a methionine-gastric lipase protein). Preferably the gastric lipase protein is a human gastric lipase protein.

The gastric lipase protein is advantageously produced by a recombinant DNA technique.

In a second aspect of the invention we provide a process for the production of a methionine-gastric lipase protein comprising producing the protein in a host organism transformed with a vector including a gene coding for the methionine-gastric lipase protein.

To obtain expression of a DNA sequence, and DNA sequence must possess a 5' ATG codon and the corresponding polypeptide therefore possesses an N-terminal methionine amino acid. As used herein the term "methionine-gastric lipase protein" denotes an authentic mammalian gastric lipase (or an authentic mammalian gastric lipase, modified or substituted to provide a functionally equivalent protein) having an N-terminal methionine amino acid residue. Preferably the methionine residue is adjacent the N-terminal amino acid of an authentic gastric lipase but may be separated therefrom by one or more amino acids provided that the protein possesses gastric lipase functional activity. Preferably the host organism is a bacterium (for example *E. coli*) or a yeast (for example *Saccharomyces cerevisiae*).

In a third aspect of the invention we provide a process for the production of a gastric lipase protein comprising producing a gastric lipase precursor protein in a host micro-organism transformed with a vector including a DNA sequence coding for the precursor protein and cleaving the precursor protein to produce the gastric lipase protein.

Preferably the gastric lipase precursor protein is a pregastric lipase protein and the host micro-organism is a host organism capable of cleaving the pregastric lipase protein to produce the gastric lipase protein. Most preferably the host micro-organism cleaves the pregastric lipase and may export the gastric lipase protein to the culture medium.

In an alternative form of the third aspect of the invention the precursor gastric lipase protein is a fusion protein comprising a heterologous protein and a gastric lipase protein. The heterologous protein may be all or a part of a protein capable of production, desirably at a high level, in the host organism. Suitable such proteins include β-galactosidase chloroamphenicol acetyl transferase (CAT) and the product of the *trpE* gene. The fusion protein preferably includes a site susceptible to selective chemical or enzymic cleavage between the heterologous protein and the gastric lipase protein. The heterologous protein may be a yeast signal sequence and the host organism may be yeast. In this preferred embodiment the yeast host organism advantageously cleaves the fusion protein to produce a mature gastric lipase protein.

In a fourth aspect of the invention we provide a pregastric lipase protein.

In a fifth aspect of the invention we provide a methionine-gastric lipase protein.

In a sixth aspect of the invention we provide a fusion protein comprising a heterologous protein and a gastric lipase protein.

In a seventh aspect of the invention we provide a DNA sequence coding for at least the amino acid sequence of a gastric lipase protein. Preferably the DNA sequence codes for a protein of the fourth, fifth, or sixth aspect of the invention.

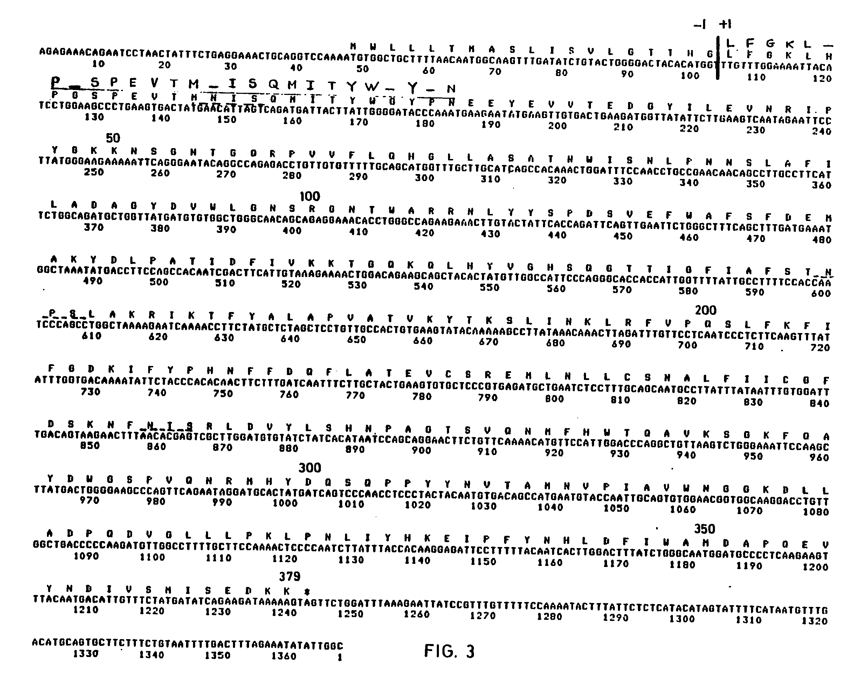

We further provide a DNA sequence coding for at least the amino acid sequence of human gastric lipase or human pre gastric lipase as shown in Figure 3 of the accompanying drawings. Preferably the DNA sequence is as shown in Figure 3.

In an eighth aspect of the invention we provide a vector including a DNA sequence of the seventh aspect of the invention. The vector is adapted for use in a given host micro-organism by the provision of suitable selectable markers, promoters and other control regions as appropriate.

In a ninth aspect of the invention we provide a host micro-organism transformed with a vector according to the eighth aspect of the invention. The host micro-organism may be any organism which may be transformed by a vector including a DNA sequence coding for a gastric lipase protein such that expression of the sequence occurs. Suitable such host organisms include bacteria (for example *E. coli*), yeasts (for example *Saccharomyces cerevisiae*) and mammalian cells in tissue culture. Preferably, where the host organism is a bacterium or a yeast the vector includes a gene coding for methionine-gastric lipase or a fusion protein, and when the host organism is a mammalian cell in tissue culture the vector preferably includes a DNA sequence coding for pregastric lipase.

In a tenth aspect of the invention we provide a pharmaceutical composition comprising a gastric lipase protein and a pharmaceutically acceptable excipient. Preferably the lipase protein is a human gastric lipase produced by a process of the second or third aspect of the invention. The pharmaceutical composition is provided for use in the treatment of lipase deficiency. Preferably the composition is formulated for oral administration.

The composition may be in unit dosage form, for example as a tablet, capsule or dragee.

To produce a unit dosage from the gastric lipase, in a suitable form, may be mixed with a solid pulverulent non-pharmaceutically active carrier such as lactose, saccharose, sorbitol mannitol, starch, cellulose derivatives or gelatine or any other such excipient. A lubricant such as magnesium stearate, calcium stearate or polyethylene glycol wax may be added. The resulting composition is compressed to form a unit dosage form. The unit dosage form may be coated with a concentrated sugar solution which may contain additives such as talc, titanium dioxide, gelatine or gum arabic. The unit dosage form may be coated with lacquer. Dyestuffs may be added to the coating to facilitate identification of the unit dosage form. Soft or hard capsules may be used to encapsulate gastric lipase as a liquid or solid preparation.

Alternatively, the gastric lipase may be formulated in a liquid form. To produce a liquid form of the preparation the gastric lipase in a suitable form may be added to a liquid carrier. The carrier may be, for example, a syrup or a suspension. The liquid form may contain colouring compounds, flavouring compounds, sweetening compounds, and/or thickening compounds.

In a further aspect of the invention we provide a process for the production of a pharmaceutical composition comprising bringing a gastric lipase protein into association with a pharmaceutically acceptable carrier. In a yet further aspect of the invention we provide a method for the treatment or lipase deficiency comprising administering an effective amount of a gastric lipase protein.

The invention also provides plasmids PGL17, pCML1 and pMG197.

In the following description a protocol is described for the production of gastric lipase using recombinant DNA technology with reference to the following drawings in which:—

Figure 1 shows an SDS polyacrylamide gel of two preparations of human gastric lipase (Lane A — purified human gastric lipase, Lane B — partially purified extract of human gastric lipase, Lane C — standard molecular weight markers),

Figure 2 shows a restriction endonuclease map of plasmid pGL17.

((a) shows the restiction map,

(b) indicates the limits of the DNA sequence shown in Figure 3 and

(c) indicates the location of the human gastric lipase protein sequence with the thick line representing the pre or signal sequence),

Figure 3 shows the DNA sequence of the coding strand of the DNA sequence encoding human pre gastric lipase and the associated amino sequence,

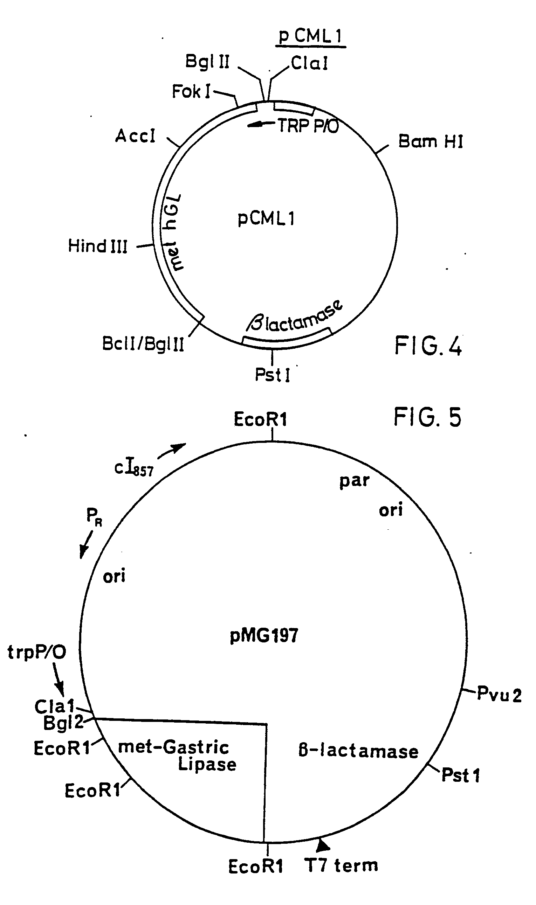

Figure 4 shows a restriction endonuclease map of plasmid pCML1,

Figure 5 shows a restriction endonuclease map of plasmid pMG197,

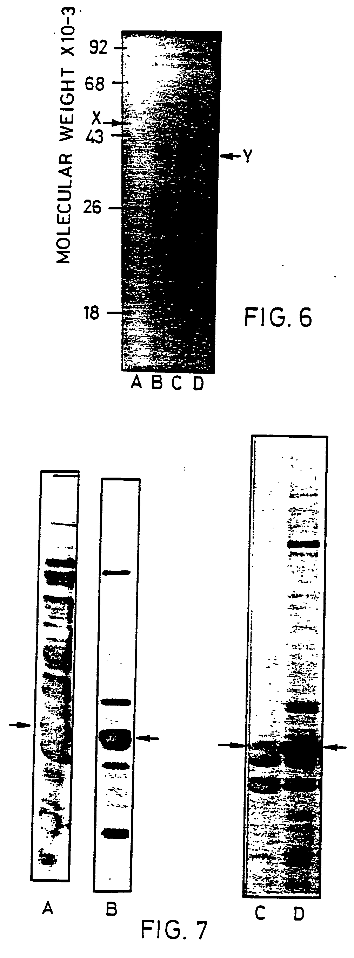

Figure 6 shows a western blot analysis of human gastric lipase produced in *E. coli* transformed with plasmid pMG197,

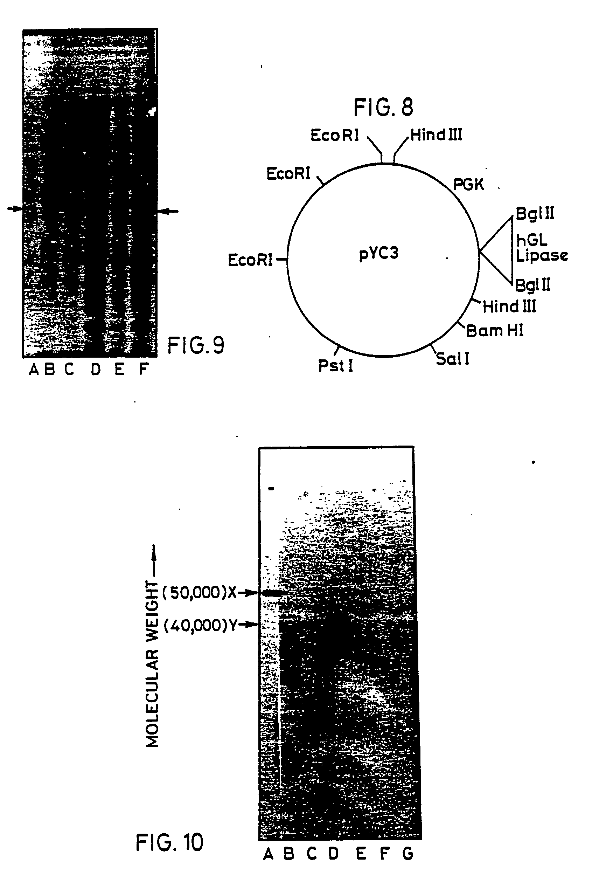

Figure 7 shows an SDS-PAGE analysis of human gastric lipase produced in *E. coli* transformed with plasmid pMG197,

Figure 8 shows a restriction endonuclease map of yeast plasmid pYC3,

Figure 9 shows a western blot analysis of human gastric lipase produced in yeast transformed with plasmid pYC3,

Figure 10 shows an SDS-PAGE analysis of human gastric lipase produced in yeast transformed with plasmid pYC3.

The strategy used was to first purify human gastric lipase from human gastric aspirates. The purified enzyme was subjected to N-terminal amino acid sequencing, structural characterisation and a polyclonal antiserum raised. The DNA sequence for this enzyme was cloned by screening a cDNA library made from human stomach tissue with a probe consisting of the highly homologous rat lingual lipase. The complete nucleic acid/protein sequence of human gastric lipase was determined. The present specification describes how this human gastric lipase clone is expressed in an appropriate micro-organism or animal cell in tissue culture to produce a recombinant human gastric lipase product.

Purification of Human Gastric Lipase from Human Stomach Aspirates

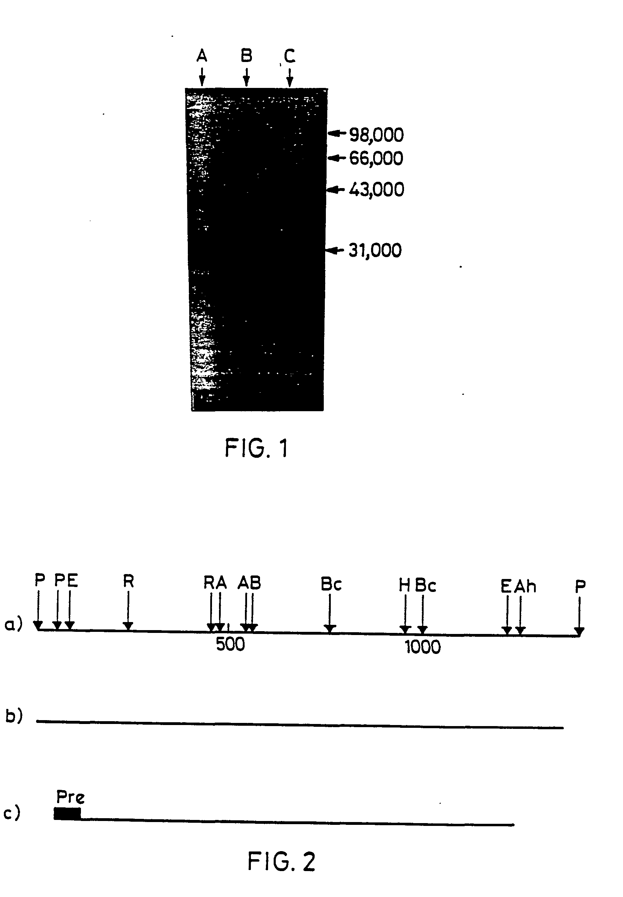

Human gastric lipase was purified from gastric aspirates by the method of Tiruppathi et al (1982) Biochim. Biophys. Acta. 712 692—697. This procedure produced pure human gastric lipase with a molecular weight of approximately 50,000 as judged by SDS PAGE. (Lammeli (1970) Nature 277 68—685), Figure 1 shows a polyacrylamide SDS gel of human gastric lipase preparations. Lane A, purified human gastric lipase (approximately 5 µg) and Lane B, a partially purified extract of human gastric aspirate (approximately 10 µg), Lane C, a series of standard molecular weight markers. The enzyme had an activity of approximately 600 lipase units per mg (unit-micromoles of free fatty acid formed per minute at 37°C).

Preparation of Polyclonal Rabbit Anti-Human Gastric Lipase Antiserum

Approximately 100 µg of a preparation of electrophoretically pure human gastric lipase isolated as described above was taken up in 1 ml complete Freund's adjuvant and injected into a rabbit. After 14 days the inoculation was repeated using incomplete Freund's adjuvant. The rabbit was bled to produce antiserum after 28—30 days and at subsequent intervals. The titre of the antiserum was determined by standard immunological procedures.

Characterisation of Authentic Human Gastric Lipase Determination of Molecular Weight

Human gastric lipase, purified to homogeneity and subjected to electrophoresis in SDS polyacrylamide gels migrated as a single band with an apparent molecular weight of approximately 50,000 (Figure 1). Gel filtration of impure human gastric lipase of Sephadex® G150 resulted in a calculated molecular weight in approximate agreement with that obtained by polyacrylamide gel electrophoresis. A molecular weight of 45,000 has been estimated by Tiruppathi et al (1982), see above, using gel filtration on Sephadex® G100. It is therefore concluded that the purified human gastric lipase is active as a monomer of approximately 50,000 molecular weight.

N-Terminal Amino Acid Sequence and Total Amino Acid Composition of Human Gastric Lipase

The N-terminal amino acid sequence of purified human gastric lipase was determined by the method of Smith, M.A. et al (1982) Biochemical Journal 207, 253—260.

N-Terminal Amino Acid Sequence of Human Gastric Lipase

```
1                                  10
Leu Phe Gly Lys Leu — Pro Thr Ser Pro Glu Val Thr Met
                  20
— Ile Ser Gln Met Ile Thr Tyr Trp
— Tyr — Asn Gln
(a dash indicates an amino acid not determined)
```

6

## TABLE 1

### Partial Amino Acid Composition of Human Gastric Lipase

| Residue | nMoles Amino Acid Eluted* | Total amino acids predicted from DNA Sequence |
|---|---|---|
| Asp + Asn | 52.4 | 48 |
| Thr | 19 | 19 |
| Ser | 28.8 | 26 |
| Glu + Gln | 38.2 | 29 |
| Pro | 24.9 | 22 |
| Gly | 29.5 | 23 |
| Ala | 28.8 | 24 |
| Val | 28.7 | 24 |
| Met | 10.6 | 9 |
| Ile | 22.2 | 22 |
| Leu | 36.0 | 33 |
| Tyr | 21.5 | 21 |
| Phe | 25.5 | 25 |
| Lys | 21.2 | 22 |
| His | 11.7 | 10 |
| Arg | 10.6 | 10 |

*Cys, Trp were not determined.

Determination of the Presence of Glycosylation in Human gastric lipase

The presence of asparagine linked N-glycosylation was established by digestion of purified human gastric lipase with Endoglycosidase H (Endo-B-N-acetylglucosaminidase H) from *streptomyces plicatus*. A 1 mg/ml solution of human gastric lipase in 50 mM sodium acetate pH 5.5, 1 mM Phenyl methyl sulfonyl fluoride, 10 µM pepstatin A containing 50 units/ml Endoglycosidase H was incubated at 37°C. Alternatively, human gastric lipase was boiled in 0.4% SDS and diluted to 0.1% SDS before incubation as above. In both cases the human gastric lipase digestion products were separated on SDS PAGE and visualised by Coomassie Blue staining. Digestion of human gastric lipase with Endoglycosidase H resulted in the generation of a series of lower molecular weight forms with a minimum molecular weight of approximately 41,000. Endoglycosidase H digestion results in the removal of N linked carbohydrate moieties from glycoproteins containing these residues. This cleavage produces an apparent lowering of the molecular weight of the deglycosylated protein. This lowering of molecular weight may be visualised by increased mobility of the deglycosylated protein on SDS PAGE. That Endoglycosidase treatment of human gastric lipase results in an apparent decrease of molecular weight from approximately 50,000 to approximately 41,000 indicates that approximately 20% of the enzyme (by weight) is composed of carbohydrate.

Cloning of Human Gastric Lipase

A DNA sequence encoding human gastric lipase was isolated from a cDNA clone bank made from mRNA prepared from a sample of human stomach tissue. Human gastric lipase clones were identified by homology with a cDNA clone of rat lingual lipase previously obtained as described in published European patent application EP—A1—0131418. (The disclosures of which are incorporated herein by reference). A freshly obtained section of human stomach wall tissue approximately 2 cm wide was stored in liquid nitrogen. The section contained complete mucosal, muscle and serosa layers. RNA was prepared by guanidinium isothiocyanate extraction of the frozen ground complete tissue (Maniatis *et al* (1982)

7

"Molecular Cloning — A Laboratory Manual". Cold Spring Harbor Laboratory). Polyadenylated RNA was isolated from this by oligo-dT cellulose chromatography (Harris, T. J. R. *et al* (1975) J. Gen. Virol *29* 299—312).

The presence of an mRNA species encoding an acid stable lipase was suggested by Northern Blot analysis (Thomas, P. S. (1980) PNAS USA, *77* 5201—5205). By this technique polyadenylated stomach RNA was separated on the basis of molecular weight by gel electrophoresis and probed with a cDNA clone of the rat lingual lipase DNA sequence labelled by nick translation (Rigby P. W. J. *et al* J. Mol. Biol. *133*, 237—251). This labelled sequence specifically hybridised with a mRNA species with an apparent size of approximately 1500 bases. This mRNA species was of a size capable of encoding a protein of the apparent size of human gastric lipase together with untranslated 5' and 3' sequences of such a message.

cDNA was prepared to the human stomach mRNA. First strands were synthesized by poly(dT) priming and elongation by AMV reverse transcriptase (Retzel, E. F. *et al* (1980) Biochemistry *19* 513—518). Second strands were synthesised by the action of RNase H, *E. coli* DNA polymerase I and *E. coli* DNA ligase as described (Gubler, V. and Hoffman, B. (1983) Gene *25*, 263—269). The double stranded cDNA was tailed at the 3' ends with poly(dT) (Villa-Komaroff *et al* (1978) PNAS USA, *75*: 3727). Tailed fragments were annealed into pBR322 which had been cleaved and poly(gD) tailed at the PstI site. These hybrids were transformed into *E. coli* DHI competent for transformation (Maniatis *et al* (1982) "Molecular Cloning — A Laboratory Manual". Cold Spring Harbor Laboratory). The transformants were screened by colony hybridisation on nitrocellulose filters (Hanahan, D. and Meselson, M (1980) *Gene 10* 63—67). The hybridisation probe was the DNA fragment containing the coding region for rat lingual lipase labelled by nick translation (Rigby, P. W. J. *et al* (1977) J. Mol. Biol. *133* 237—252).

Putative human gastric lipase clones were mapped for restriction endonuclease cleavage sites (Figure 2) and subjected to DNA sequencing (Sanger, F. S., *et al* (1977) PNAS USA *74* 5463—5467; Smith, A. J. H. (1980) "Methods in Enzymology" Academic Press *65* 560—580) using a synthetic single-stranded oligodeoxyribonucleotide primer which hybridised to a region just 3' to the cloned segment. Clones were shown to encode the lipase by sequence homology with the rat lingual lipase cDNA sequence and comparison of the predicted sequence from the cDNA clones with the N-terminal amino acid sequence of native human gastric lipase isolated from stomach aspirate (Table 1 and Figure 3). One clone was identified (pGL17), approximately 1450 bp long containing the entire coding sequence for the gastric prelipase. The 5' end of the clone was shown to be within 20 nucleotides of 5' terminal nucleotide of the message. This was demonstrated by the sequence obtained from the primer extension. In this technique a synthetic oligodeoxyribonucleotide primer was hybridised specifically to a region of the human gastric lipase mRNA encoding the N-terminal protein sequence. This primer was extended to the 5' end of the mRNA and the sequence determined. A restriction endonuclease map of pGL17 was constructed and is shown in Figure 2, line a. Numbers below this line relate to the base numbering given in Figure 3. Figure 2, line b indicates the limits of the DNA sequence provided in Figure 3. The location of the human gastric lipase protein sequence is shown in Figure 2, line c. The thick line labelled "pre" refers to the location of the "pre" or signal sequence of human gastric lipase. (In Figure 2 the restriction site abbreviations are as follows: P=PstI, E=EcoRV, R=EcoRI, A=AluI, B-BalI, Bc=BclI, and Ah=AhaIII).

The DNA sequence of the coding strand of the pre human gastric lipase gene is shown in Figure 3. Numbers below the DNA sequence represent the base number. Base 1 is the first nucleotide of the cloned human gastric lipase sequence in pGL17. An "*" indicates the stop codon TAG which is followed by a 3' untranslated region. Letters immediately above the bases represent the derived amino sequence, using the conventional single letter amino acid code (i.e. A=alanine, R=arginine, N=asparagine, D=aspartic acid, C=cysteine, E=glutamic acid, Q=glutamine, G=glycine, H=histidine, I=isoleucine, L=leucine, K=lysine, M=methionine, F=phenylalanine, P=proline, S=serine, T=threonine, W=tryptophan, Y=tyrosine and V=valine).

Underlined letters above the derived amino acid sequence represent the N-terminal amino acid sequence obtained directly from purified human gastric lipase. Spaces in the directly obtained amino sequence represent undetermined amino acids. Amino acids −19 to −1 represent a putative signal sequence and +1 to 379, the amino acid sequence of the mature DNA sequence. Broken underlining indicates the potential glycosylation sequence. The amino acid sequence predicted from the DNA sequence indicates that mature human gastric lipase consists of a 379 amino acid protein. The predicted molecular weight of this mature protein is 43, 162 which is in close agreement with the molecular weight determined for the deglycosylated enzyme by SDS PAGE. The total amino acid composition of the mature enzyme produced from the DNA sequence is compared with that obtained directly from the isolated protein in *Table 1*. Mature human gastric lipase contains 3 potential sites for glycosylation (of the general form X Asn X Thr or Ser). Human gastric lipase is 70 amino acids shorter than porcine pancreatic lipase and bears little sequence homology or amino acid composition similarity to this enzyme. However, close homology does exist between human gastric lipase and porcine pancreatic lipase in the region of the essential serine-152 of porcine pancreatic lipase. The serine is thought to participate in the interfacial fixation of pancreatic lipase to lipid (Guidoni, A. *et al* 1981, Biochim. Biophys. Acta. *660*, 148—150) and reacts with micellar diethyl-p-nitrophenyl phosphate (Rouard, M. *et al* 1978, Biochim. Biophys. Acta. *530*, 227—235). It is present in the sequence: 152 Gly- His- Ser- Leu- Gly in Porcine Pancreatic Lipase and in a closely equivalent position in the primary amino acid sequence:

153

Gly-His-Ser-Gly in Human Gastric Lipase

Another point of similarity, close to this essential serine residue, is the single glycosylation position in porcine pancreatic lipase (Asn-166) which appears to be present in Asn-166 in human gastric lipase.

Mature human gastric lipase has a striking amino acid sequence homology (approximately 76%) with rat lingual lipase (see published European patent application EP—A1—00131418). However, the amino acid sequence of the signal sequences of human gastric lipase and rat lingual lipase do show certain differences. Only the 5 N-terminal and 2 C-terminal amino acid residues of the signal sequences are homologous. Human gastric lipase contains one less cys residue and one less potential glycosylation site than rat lingual lipase. The retained cys residues and glycosylation sites are in virtually equivalent positions in the primary amino sequence of human gastric lipase and rat lingual lipase.

Expression of human gastric lipase in (i) E. coli (ii) Yeast (iii) Tissue cultured animal cells (i) E. coli

A plasmid vector for the expression of mature methionine-human gastric lipase (hereinafter referred to simply as human gastric lipase) was constructed based on the dual replication origin temperature inducible vector system described in published European patent application EP—A1—0121386. (The disclosures of which are incorporated herein by reference). This plasmid was constructed using the complete prelipase DNA sequence on a PstI to Aha III DNA fragment of the cloned DNA sequence. The 3' end of the DNA sequence was isolated as an AccI to BglII fragment, and the 5' end as a FokI to AccI fragment (see Fig. 4). To this a pair of linkers were added at the 5' FokI end. These oligonucleotides reconstructed the 5' end of the lipase gene, added an ATG start site, provided a BglII site in the Shine-Dalgarno-ATG region and provided a ClaI site for cloning into pCT54 (described in Emtage *et al* Proc. Natl. Acad. Sci. (1983) 3671—3675). pCML1 was constructed by digestion of pCT54 with ClaI and BclI followed by a three way ligation carried out as follows:—

(1) ClaI/BclI vector

(2) ClaI/AccI 5' end

(3) AccI/BglII 3' end

This yielded pCMLI with a Shine Dalgarno ATG distance of 14 nucleotides.

A restriction endonuclease map of plasmid pCML1 is shown in Fig. 4. The nucleotide sequence in the region of the *trp* promoter and start of the DNA sequence encoding human gastric lipase is shown below:

ClaI BglII

ACGTAAAA*AGGG*TATCGATAGATCTA*TGTTGTTT*.....

Shine-Dalgarno            Met Leu Phe........

sequence                human gastric lipase

                    DNA sequence

The plasmid used for expression of human gastric lipase in *E. coli* was termed pMG197. This plasmid was based on pMG165, a dual replication origin vector described in published European patent application EP—AI-0121386. pMG197 was constructed by digestion of pCML1 with BamHI and PstI and the fragment bearing the human gastric lipase DNA sequence isolated. pMG171 (related to pMG165 as described in published European patent application EP—A1—0121386) was also digested with BamHI and PstI and the human gastric lipase DNA sequence containing fragment inserted to form pMG197 (Fig. 5). This plasmid was isolated and transformed into *E. coli* E103(S).

*E coli* containing pMG197 was grown as described in published European patent application EP—A1—0121386 in a 10 litre fermentation vessel, the cells harvested by centrifugation and stored at −20°C.

A "Western blot" analysis on total proteins present in E103(S)/pMG197 was carried out as described by Burnette, (Burnette, W. N. (1981) Anal. Biochem. 112, 195—203). In this analysis total proteins were separated by SDS-PAGE and transferred to a nitrocellulose membrane. Human gastric lipase was detected using polyclonal antiserum to natural human gastric lipase and the complex labelled with $^{125}$I-Protein A (Fig. 6).

The arrow labelled X (lane A) indicates the position of migration of natural human gastric lipase. The arrow labelled Y indicates the position of a novel protein produced in E103(S)/pMG197 after a temperature induction carried out as described in published European patent application EP—A1—0121386. Lanes B and C correspond to total proteins extracted from cells harvested 3 hours and 2 hours after temperature induction. Uninduced cells are shown in Lane D. This analysis indicated that E103(S)/pMG197 expressed human gastric lipase as a prominent protein migrating with an apparent molecular weight of approximately 38,000. The discrepancy between the apparent molecular weights of natural human gastric lipase (approx. 50,000) and recombinant human lipase (approx. 38,000) could be due to the inability of *E. coli* to carry out glycosylation. Unglycosylated human gastric lipase has a molecular weight of 43,162 as predicted by amino acid sequence derived from the DNA sequence of the cloned gene.

Human gastric lipase was partially purified from *E. coli* and solubilised as described below. 10 g of frozen cell paste of E103(S)/pMG197 was resuspended in 50 ml of 50 mM Tris pH8, 50mM NaCl, 1mM EDTA and 0.1 mM PMSF. All manipulations were carried out at 4°C unless indicated otherwise. Suspended cells were passed three times through a French Press operating at 1,500 psi. The suspension of broken cells was centrifuged at 12,000 for 5 minutes. Samples of the supernatant were retained for analysis and the pellet fraction (containing the human gastric lipase product in an insoluble form) resuspended in 50mM Tris pH8, 10mM EDTA and 0.5% Triton® X—100. The resuspended pellet fraction containing the insoluble human gastric lipase product was recentrifuged as described above. Insoluble human gastric lipase was solubilised by urea or alkali in a manner similar to that described in British Patent Specification GB2100737B and in British patent applications GB2129810A and GB2138004A. Insoluble human gastric lipase was dissolved in 50mM Tris pH8, 8M urea at room temperature at a final protein concentration of approximately 1 mg/ml. Denaturant was removed by dialysis against a solution of 50 mM sodium carbonate/bicarbonate buffer at pH 10.7. Precipitated protein was removed by centrifugation.

An SDS-PAGE analysis of human gastric lipase expression and solubilisation is shown in Fig. 7. Arrows indicate the expressed human gastric lipase protein. Total proteins from E103(S)/pMG197 are shown in Lane A. Quantitative gel scanning indicated that human gastric lipase was expressed as approximately 8% of total *E. coli* proteins. Lane B shows the composition of the proteins present in the washed insoluble cell extract. Human gastric lipase constituted a major proportion of the insoluble protein present in the pellets produced by centrifugation. Lanes C and D show the insoluble and soluble proteins, respectively, present after removal of urea by dialysis. This indicates that human gastric lipase constitutes the major protein present in the soluble extract.

Employing broadly similar techniques, expression of genes coding for a human pregastric lipase or for a fusion protein including gastric lipase may be achieved. See for example the disclosures of published European patent application EP—A1—131363 for a description of the preparation of vectors capable of expressing a DNA sequence coding for chloramphenicol acetyl transferase fusion proteins.

(ii) Expression of human gastric lipase in yeast

Plasmid vectors for the expression of methionine human gastric lipase were constructed based on plasmid pMA91 (also known by the designation pMA3013) as described in the published european patent application EP—A2—0073653. These vectors contain the yeast phosphoglycerate kinase (PGK) promoter and the PGK gene 3' end flanking sequences sandwiching the methionine-human gastric lipase DNA sequence. A plasmid pMB1 (not shown) was constructed by insertion of a BglII fragment containing the entire pre human gastric lipase DNA sequence. The plasmid pYC3 (Fig. 8) was constructed by removal of a BglII to AccI fragment frm pMBI containing the 3' end of the lipase DNA sequence and ligated to the BglII to AccI fragment of the 5' end of the gene obtained from pCML1 (described above). This was inserted into the BglII site of pMA3013 to form pYC3. The plasmid pYC3 was transformed into the diploid strain MD50 and the haploid MD40/4C and transformants grown up in nitrogen based medium as described in the published European patent application EP—A2—0073653. Harvested cells were stored at −20°C.

A frozen slurry of yeast cells MD50 containing pYC3 was resuspended in 50 mM Tris pH 7.5, 1 mM EDTA. All operations were carried out at 4°C. Cells were broken in a French Press by three passes at a pressure of 1,500 psi. Residual intact cells were removed by centrifugation at 800 g for 5 minutes. The supernatant, termed "total extract", was centrifuged at 20,000 g for 5 minutes to remove cell debris. The clear supernatant, termed "soluble extract" was retained for protein analysis by SDS-PAGE. The pelleted cell debris were washed in the above buffer by resuspension and recentrifugation. Washed cell debris were resuspended in an equal volume of the above buffer and samples were taken for SDS-PAGE. The above procedures were repeated on yeast DM40/4C cells bearing plasmid pYC3 and, as a control, yeast MD50 containing an equivalent plasmid to pYC3 but without the human gastric lipase DNA sequence. SDS-PAGE analysis of total protein extracts from these cells is seen in Fig. 9. Proteins were visualised by Coomassie blue staining. An arrow indicates the position of migration of recombinant human gastric lipase. Lanes A and B show the washed cell debris and soluble extract fractions respectively from yeast MD40/4C containing the control plasmid. No protein corresponding to human gastric lipase is visible. Lanes C and D represents the debris and soluble extract fractions respectively from yeast MD/40 containing pYC3. Similarly, lanes E and F contain equivalent fractions from yeast MD50. A prominent protein is seen in the debris fraction of both yeast MD40/4C and MD50 containing pYC3 migrating in the expected position for recombinant human gastric lipase. Quantification of this protein by gel scanning indicated an expression level of 1%—3% of total protein (depending on fermentation batch).

A Western blot analysis was carried out on proteins present in yeast MD50/pYC3 (Fig. 10). The arrow labelled X (Lane A) indicates the position of migration of natural human gastric lipase. The arrow labelled Y indicates the position of the protein produced in MD50/pYC3, also indicated by an arrow in Fig. 10. Lanes B, C and D represent, respectively, an analysis of: total proteins; soluble extract and the cell debris fraction. A prominent band of human gastric lipase is seen migrating with an apparent molecular weight of approximately 40,000 in the total extract and insoluble debris fraction. Virtually no human gastric lipase was detectable in the soluble extract fraction. Lanes E, F and G represent analysis of the total proteins, soluble extract and debris fraction of yeast MD50 containing an equivalent plasmid to pYC3 but without the human gastric lipase gene. No human gastric lipase was detectable in these control cells. This analysis confirmed that yeast MD50/pYC3 expressed human gastric lipase. The Western blot analysis was repeated on yeast MD40/4C containing pYC3 with similar results. Again, a discrepancy is seen between the apparent molecular weights of natural human gastric lipase (approx. 50,000) and recombinant human gastric lipase (approx. 40,000). This may be due to an inability of yeast to carry out glycosylation of human gastric lipase produced intracellularly in yeast. The presence of lipolytically active human gastric lipase in yeast was shown by assay of total cell extracts and the soluble and insoluble fractions in the human gastric lipase activity assay. Soluble and insoluble extracts of MD50/pYC3, MD40(4C) pYC3 and MD50 control were made as described above with a "citrate-phosphate" buffer (50 mM sodium phosphate brought to pH 5.4 with 50 mM citric acid) substituted for 0.05M Tris, 1 mM EDTA. 25 µl samples were taken from assay, as described above, using a triolein substrate at 37°C. The activity of recombinant human gastric lipase was compared with natural human gastric lipase as shown in Table II. Lipolytic activity was detected in the total extract and soluble and insoluble fractions. This activity was not present in control cells lacking the DNA sequence encoding human gastric lipase. From the lipase activity present in the total homogenate and an human gastric lipase expression level of approximately 3% total protein it can be calculated that approximately 5% and 18% of human gastric lipase produced in yeast MD40/4C and MD50 respectively was catalytically active.

## Table II

### Lipolytic Activity of human gastric lipase Expressed in Yeast

| Yeast Strain/Plasmid | Total extract | Activity* Soluble Fraction | Insoluble Fraction |
|---|---|---|---|
| MD40/4C/pYC3 | 14,967 | 8,250 | 8,792 |
| MD50/pYC3 | 27,445 | 14,394 | 12,040 |
| Control, MD50 | 2,677 | 1,761 | 1,374 |
| Buffer Blank | 3,630 | - | - |

*cpm, production of $^{14}$oleic acid from $^{14}$C-triolein; reaction conditions as described in the text. 1ug natural human gastric lipase was equivalent to 7,995 cpm in this assay.

Employing broadly similar techniques expression of DNA sequences coding for human pregastric lipase or for a fusion protein including gastric lipase may be achieved.

(iii) Tissue Cultured Animal Cells

Plasmid vectors for the expression of human pregastric lipase are constructed based on vectors described by Pavlakis, G. N. and Hamer, D. H. (1983) Proc. Nat. Aca. Sci. USA 80, 397—401). These vectors contain metallothionine gene promoters and express prehuman gastric lipase. The enzyme produced in this system is secreted through the cellular membrane and is assayed in, and purified from the tissue culture medium as described above. The tissue cultured animal cells may possess the processing functions necessary to produce mature gastric lipase.

It will of course be understood that the present invention has been described above purely by way of example and modifications of detail can be made within the scope of the invention.

**Claims**

1. A gastric lipase protein comprising the amino acid sequence of a mammalian gastric lipase protein secreted by the gastric mucosa or a modified or substituted form thereof which is a functionally equivalent protein, for use in the treatment of lipase deficiency.

2. A gastric lipase protein according to claim 1 wherein the gastric lipase protein comprises the amino acid sequence of human gastric lipase protein or a modified or substituted form thereof which is a functionally equivalent protein.

3. A process for the production of a methionine-gastric lipase protein comprising the amino acid sequence of a mammalian gastric lipase protein secreted by the gastric mucosa or a modified or substituted form thereof which is a functionally equivalent protein comprising the step of producing the protein in a host micro-organism transformed with a vector including a DNA sequence coding for the methionine-gastric lipase protein.

4. A process for the production of a gastric lipase protein comprising the amino acid sequence of a mammalian gastric lipase protein secreted by the gastric mucosa or a modified or substituted form thereof which is a functionally equivalent protein comprising the step of producing a gastric lipase precursor protein in a host micro-organism transformed with a vector including a DNA sequence coding for the gastric lipase precursor protein and cleaving the precursor protein to produce the gastric lipase protein.

5. A process according to claim 4 wherein the gastric lipase precursor protein is a pregastric lipase protein and the host micro-organism is a host organism capable of cleaving the pregastric lipase to produce the gastric lipase protein.

6. A process according to claim 5 wherein the host organism is a mammalian cell in tissue culture.

7. A process according to claim 4 wherein the gastric lipase precursor protein comprises a heterologous protein and a gastric lipase protein.

8. A pregastric lipase protein comprising the amino acid sequence of a mammalian gastric lipase protein secreted by the gastric mucosa or a modified or substituted form thereof which is a functionally equivalent protein.

9. A methionine-gastric lipase protein comprising the amino acid sequence of a mammalian gastric lipase protein secreted by the gastric mucosa or a modified or substituted form thereof which is a functionally equivalent protein.

10. A DNA sequence coding for a protein comprising the amino acid sequence of a mammalian gastric lipase protein secreted by the gastric mucosa or a modified or substituted form thereof which is a functionally equivalent protein.

11. A mammalian gastric lipase protein for use in the treatment of lipase deficiency comprising the following amino acid sequence:

```
LFGKLHPGSPEVTMNISQMITYWGYPNEEYEVVTEDGYILEVNRIPYGKKNSGN
TGQRPVVFLQHGLLASATNWISNLPNNSLAFILADAGYDVWLGNSRGNTWARRN
LYYSPDSVEFWAFSFDEMAKYDLPATIDFIVKKTGQKQLHYVGHSQGTTIGFIA
FSTNPSLAKRIKTFYALAPVATVKYTKSLINKLRFVPQSLFKFIFGDKIFYPHN
FFDQFLATEVCSREMLNLLCSNALFIICGFDSKNFNISRLDVYLSHNPAGTSVQ
NMFHWTQAVKSGKFQAYDWGSPVQNRMHYDQSQPPYYNVTAMNVPIAVWNGGKD
LLADPQDVGLLLPKLPNLIYHKEIPFYNHLDFIWAMDAPQEVYNDIVSMISEDKK
```

12. A pharmaceutical composition comprising a gastric lipase protein comprising the amino acid sequence of a mammalian gastric lipase protein secreted by the gastric mucosa or a modified or substituted form thereof which is a functionally equivalent protein, and a pharmaceutically acceptable excipient.

**Patentansprüche**

1. Gastrolipaseprotein mit der Aminosäuresequenz eines durch Magenschleimhaut abgesonderten Säugetier-Gastrolipaseproteins oder einer modifizierten oder substituierten Form desselben, die ein funktionell äquivalentes Protein darstellt, zur Behandlung von Lipasedefizienz.

2. Gastrolipaseprotein nach Anspruch 1, welches die Aminosäuresequenz von humanen Gastrolipaseproteins oder einer modifizierten oder substituierten Form desselben, die ein funktionell äquivalentes Protein darstellt, enthält.

3. Verfahren zur Herstellung eines Methionin-Gastrolipaseproteins mit der Aminosäuresequenz eines durch Magenschleimhaut abgesonderten Säugetier-Gastrolipaseproteins oder einer modifizierten oder substituierten Form desselben, die ein funktionell äquivalentes Protein darstellt, welches Verfahren den Schritt der Produktion des Proteins in einem Wirktsmikroorganismus, der mit einem Vektor transformiert ist, der eine für das Methionin-Gastrolipaseprotein codierende DNA-Sequenz enthält, umfaßt.

4. Verfahren zur Herstellung eines Gastrolipaseproteins mit der Aminosäuresequenz eines durch Magenschleimhaut abgesonderten Säugetier-Gastrolipaseproteins oder einer modifizierten oder substituierten Form desselben, die ein funktionell äquivalentes Protein darstellt, welches Verfahren den Schritt der Produktion eines Gastrolipase-Precursorproteins in einem Wirtsmikroorganismus, der mit einem Vektor transformiert ist, der eine für das Gastrolipase-Precursorprotein codierende DNA-Sequenz enthält, und die Spaltung des Precursorproteins zur Gewinnung des Gastrolipaseproteins umfaßt.

5. Verfahren nach Anspruch 4, bei welchem das Gastrolipase-Precursorprotein ein pregastrisches Lipaseprotein und der Wirtsmikroorganismus ein Wirtsorganismus ist, der zur Spaltung der pregastrischen Lipase zur Gewinnung des Gastrolipaseproteins befähigt ist.

6. Verfahren nach Anspruch 5, bei welchem der Wirtsorganismus eine Säugetierzelle in Gewebskultur ist.

7. Verfahren nach Anspruch 4, bei welchem das Gastrolipase-Precursorprotein ein heterologes Protein und ein Gastrolipaseprotein umfaßt.

8. Pregastrisches Lipaseprotein mit der Aminosäuresequenz eines durch Magenschleimhaut abgesonderten Säugetier-Gastrolipaseproteins oder einer modifizierten oder substituierten Form desselben, die ein funktionell äquivalentes Protein darstellt.

9. Methionin-Gastrolipaseprotein mit der Aminosäuresequenz eines durch Magenschleimhaut

# EP 0 191 061 B1

abgesonderten Säugetier-Gastrolipaseproteins oder einer modifizierten oder substituierten Form desselben, die ein funktionell äquivalentes Protein darstellt.

10. DNA-Sequenz, die für ein Protein mit der Aminosäuresequenz eines durch Magenschleimhaut abgesonderten Säugetier-Gastrolipaseproteins oder einer modifizierten oder substituierten Form desselben, die ein funktionell äquivalentes Protein darstellt, codiert.

11. Säugetier-Gastrolipaseprotein zur Verwendung bei der Behandlung von Lipasedefizienz, das die folgende Aminosäuresequenz enthält:

```
LFGKLHPGSPEVTMNISQMITYWGYPNEEYEVVTEDGYILEVNRIPYGKKNSGN
TGQRPVVFLQHGLLASATNWISNLPNNSLAFILADAGYDVWLGNSRGNTWARRN
LYYSPDSVEFWAFSFDEMAKYDLPATIDFIVKKTGQKQLHYVGHSQGTTIGFIA
FSTNPSLAKRIKTFYALAPVATVKYTKSLINKLRFVPQSLFKFIFGDKIFYPHN
FFDQFLATEVCSREMLNLLCSNALFIICGFDSKNFNISRLDVYLSHNPAGTSVQ
NMFHWTQAVKSGKFQAYDWGSPVQNRMHYDQSQPPYYNVTAMNVPIAVWNGGKD
LLADPQDVGLLLPKLPNLIYHKEIPFYNHLDFIWAMDAPQEVYNDIVSMISEDKK
```

12. Pharmazeutische Zusammensetzung, die ein Gastrolipaseprotein mit der Aminosäuresequenz eines durch Magenschleimhaut abgesonderten Säugetier-Gastrolipaseproteins oder eine modifizierten oder substituierten Form desselben, die ein funktionell äquivalentes Protein darstellt, sowie einen pharmazeutisch verwendbaren Träger enthält.

## Revendications

1. Protéine lipase gastrique comprenant la séquence d'acides aminés d'une protéine lipase gastrique de mammifère sécrétée par la muqueuse gastrique ou d'une forme modifiée ou substituée de celle-ci qui est une protéine fonctionnellement équivalente, utilisable dans le traitement du déficit en lipase.

2. Protéine lipase gastrique selon la revendication 1, dans laquelle la protéine lipase gastrique comprend la séquence d'acides aminés d'une protéine lipase gastrique humaine ou d'une forme modifiée ou substituée de celle-ci qui est une protéine fonctionnellement équivalente.

3. Procédé de production d'une protéine lipase gastrique de méthionine comprenant la séquence d'acides aminés d'une protéine lipase gastrique de mammifère sécrétée par la muqueuse gastrique ou d'une forme modifiée ou substituée de celle-ci qui est une protéine fonctionnellement équivalente, comprenant l'étape de production de la protéine dans un micro-organisme hôte transformé avec un vecteur renfermant une séquence d'ADN codant pour la protéine lipase gastrique de méthionine.

4. Procédé de production d'une protéine lipase gastrique comprenant la séquence d'acides aminés d'une protéine lipase gastrique de mamifère sécrétée par la muqueuse gastrique ou d'une forme modifiée ou substituée de celle-ci qui est une protéine fonctionnellement équivalente, comprenant l'étape de production d'une protéine précurseur de lipase gastrique dans un micro-organisme hôte transformé avec un vecteur renfermant une séquence d'ADN codant pour la protéine précurseur de lipase gastrique et clivant la protéine précurseur pour produire la protéine lipase gastrique.

5. Procédé selon la revendication 4, dans lequel la protéine précurseur de lipase gastrique est une protéine lipase prégastrique et le micro-organisme hôte est un organisme hôte capable de cliver la lipase prégastrique pour produire la protéine lipase gastrique.

6. Procédé selon la revendication 5, dans lequel l'organisme hôte est une cellule de mammifère en culture tissulaire.

7. Procédé selon la revendication 4, dans lequel la protéine précurseur de lipase gastrique comprend une protéine hétérologue et une protéine lipase gastrique.

8. Protéine lipase prégastrique comprenant la séquence d'acides aminés d'une protéine lipase gastrique de mammifère sécrétée par la muqueuse gastrique ou d'une forme modifiée ou substituée de celle-ci qui est une protéine fonctionnellement équivalente.

9. Protéine lipase gastrique de méthionine comprenant la séquence d'acides aminés d'une protéine lipase gastrique de mammifère sécrétée par la muqueuse gastrique ou d'une forme modifiée ou substituée de celle-ci qui est une protéine fonctionnellement équivalente.

10. Séquence d'ADN codant pour une protéine comprenant la séquence d'acides aminés d'une protéine lipase gastrique de mammifère sécrétée par la muqueuse gastrique ou d'une forme modifiée ou substituée de celle-ci qui est une protéine fonctionnellement équivalente.

13

11. Protéine lipase gastrique de mammifère utilisable dans le traitement du déficit en lipase comprenant la séquence d'acides aminés suivante:

```
LFGKLHPGSPEVTMNISQMITYWGYPNEEYEVVTEDGYILEVNRIPYGKKNSGN
TGQRPVVFLQHGLLASATNWISNLPNNSLAFILADAGYDVWLGNSRGNTWARRN
T.YYSPDSVEFWAFSFDEMAKYDLPATIDFIVKKTGQKQLHYVGHSQGTTIGFIA
FSTNPSLAKRIKTFYALAPVATVKYTKSLINKLRFVPQSLFKFIFGDKIFYPHN
FFDQFLATEVCSREMLNLLCSNALFIICGFDSKNFNISRLDVYLSHNPAGTSVQ
NMFHWTQAVKSGKFQAYDWGSPVQNRMHYDQSQPPYYNVTAMNVPIAVWNGGKD
LLADPQDVGLLLPKLPNLIYHKEIPFYNHLDFIWAMDAPQEVYNDIVSMISEDKK
```

12. Composition pharmaceutique comprenant une protéine lipase gastrique comprenant la séquence d'acides aminés d'une protéine lipase gastrique de mammifère sécrétée par la muqueuse gastrique ou d'une forme modifiée ou substituée de celle-ci qui est une protéine fonctionnellement équivalente, et un excipient pharmaceutiquement acceptable.

FIG. 1

FIG. 2

EP 0 191 061 B1

```
                                      M W L L L T M A S L I S V L G T I H G| L F G K L —
AGAGAAACAGAATCCTAACTATTTCTGAGGAAACTGCAGGTCCAAAATGTGGCTGCTTTTTAACAATGGCAAGTTTGATATCTGTACTGGGGACTACACATGG|TTGTTTGGAAAATTACA
      10        20        30        40        50        60        70        80        90       100       110       120
```

```
  P _ S P E V T M _ I S Q M I T Y W _ Y _ N
  P G S P E V T H N I S Q M I T Y W U Y P H E E Y E V V T E D G Y I L E V N R I P
TCCTGGAAGCCCTGAAGTGACTATGAACATTAGTCAGATGATTACTTATTGGGGATACCCAAATGAAGAATATGAAGTTGTGACTGAAGATGGTTATATTCTTGAAGTCAATAGAATTCC
      130       140       150       160       170       180       190       200       210       220       230       240
     50
```

```
  Y G K K N S G N T G Q R P V V F L Q H G L L A S A T H W I S N L F N N S L A F I
TTATGGGAAGAAAAATTCAGGGAATACAGGCCAGAGACCTGTTGTGTTTTTGCAGCATGGTTTGCTTGCATCAGCCACAAACTGGATTTCCAACCTGCCGAACAACAGCCTTGCCTTCAT
      250       260       270       280       290       300       310       320       330       340       350       360
                                            100
```

```
  L A D A G Y D V W L G N S R G N T W A R R N L Y Y S P D S V E F W A F S F D E M
TCTGGCAGATGCTGGTTATGATGTGTGGCTGGGCAACAGCAGAGGAAACACCTGGGCCAGAAGAAACTTGTACTATTCACCAGATTCAGTTGAATTCTGGGCTTTCAGCTTTGATGAAAT
      370       380       390       400       410       420       430       440       450       460       470       480
```

```
  A K Y D L P A T I D F I V K K T G Q K Q L H Y V G H S Q G T T I G F I A F S T N
GGCTAAATATGACCTTCCAGCCACAATCGACTTCATTGTAAAGAAAACTGGACAGAAGCAGCTACACTATGTTGGCCATTCCCAGGGCACCACCATTGGTTTTATTGCCTTTTCCACCAA
      490       500       510       520       530       540       550       560       570       580       590       600
```

```
  P S L A K R I K T F Y A L A P V A T V K Y T K S L I N K L R F V P Q S L F K F I
TCCCAGCCTGGCTAAAAGAATCAAAAACCTTCTATGCTCTAGCTCCTGTTGCCACTGTGAAGTATACAAAAAAGCCTTATAAACAAACTTAGATTTGTTCCTCAATCCCTCTTCAAGTTTAT
      610       620       630       640       650       660       670       680       690       700       710       720
                                                                          200
```

```
  F G D K I F Y P H N F F D Q F L A T E V C S R E M L N L L C S N A L F I I C G F
ATTTGGTGACAAAATATTCTACCCACACAACTTCTTTGATCAATTTCTTGCTACTGAAGTGTGCTCCCGTGAGATGCTGAATCTCCTTTGCAGCAATGCCTTATTTATAATTTGTGGATT
      730       740       750       760       770       780       790       800       810       820       830       840
```

```
  D S K N F N I S R L D V Y L S H N P A G T S V Q N M F H W T Q A V K S G K F Q A
TGACAGTAAGAACTTTAACACGAGTCGCTTGGATGTGTATCTATCACATAATCCAGCAGGAACTTCTGTTCAAAACATGTTCCATTGGACCCAGGCTGTTAAGTCTGGGAAATTCCAAGC
      850       860       870       880       890       900       910       920       930       940       950       960
                                            300
```

```
  Y D W G S P V Q N R M H Y D Q S Q P P Y Y N V T A M N V P I A V W N G G K D L L
TTATGACTGGGGAAGCCCAGTTCAGAATAGGATGCACTATGATCAGTCCCAACCTCCCTACTACAATGTGACAGCCATGAATGTACCAATTGCAGTGTGGAACGGTGGCAAGGACCTGTT
      970       980       990      1000      1010      1020      1030      1040      1050      1060      1070      1080
```

```
  A D P Q D V G L L L P K L P N L I Y H K E I P F Y N H L D F I W A M D A P Q E V
GGCTGACCCCCAAGATGTTGGCCTTTTGCTTCCAAAACTCCCCAATCTTATTTACCACAAGGAGATTCCTTTTTACAATCACTTGGACTTTATCTGGGCAATGGATGCCCCTCAAGAAGT
     1090      1100      1110      1120      1130      1140      1150      1160      1170      1180      1190      1200
                                                                          350
```

```
  Y N D I V S H I S E D K K *
TTACAATGACATTGTTTCTATGATATCAGAAGATAAAAAGTAGTTCTGGATTTAAAGAATTATCCGTTTGTTTTTCCAAAATACTTTATTCTCTCATACATAGTATTTTCATAATGTTTG
     1210      1220      1230      1240      1250      1260      1270      1280      1290      1300      1310      1320
     379
```

```
ACATGCAGTGCTTCTTTCTGTAATTTTGACTTTAGAAATATATTGGC
     1330      1340      1350      1360       1
```

FIG. 3

FIG. 4

FIG. 5

FIG. 6

FIG. 7

4

FIG. 8

FIG. 9

A B C D E F

EcoRI    HindIII

EcoRI                    PGK

                         BglII
                         hGL
pYC3                     Lipase
EcoRI
                         BglII

                         HindIII

                         Bam HI

PstI            SalI

FIG. 10

MOLECULAR WEIGHT ⟶

(50,000) X ⟶

(40,000) Y ⟶

A B C D E F G